# EUROPÄISCHE PATENTSCHRIFT

(11) **EP 3 056 934 B1**
(45) Veröffentlichungstag und Bekanntmachung des Hinweises auf die Patenterteilung: **04.11.2020**
(21) Anmeldenummer: 16151830.3
(22) Anmeldetag: 19.01.2016
(51) Int. Cl.: G02B 23/24, G01B 11/25

(54) **MESSKOPF EINER ENDOSKOPISCHEN VORRICHTUNG UND VERFAHREN ZUR INSPEKTION UND MESSUNG EINES OBJEKTES**
MEASURING HEAD OF AN ENDOSCOPIC DEVICE AND METHOD OF INSPECTING AND MEASURING AN OBJECT
TETE DE MESURE D'UN DISPOSITIF ENDOSCOPIQUE ET PROCEDE D'INSPECTION ET MESURE D'UN OBJET

(30) Priorität: 29.01.2015 DE 102015201561
(43) Veröffentlichungstag der Anmeldung: 17.08.2016
(73) Patentinhaber: Rolls-Royce Deutschland Ltd & Co KG, 15827 Blankenfelde-Mahlow (DE)
(72) Erfinder: BAUER, Joachim, 15827 Blankenfelde-Mahlow (DE); SCHRADER, Sigurd, 15827 Blankenfelde-Mahlow (DE); BURGER, Martin, 15827 Blankenfelde-Mahlow (DE); PULWER, Silvio, 15827 Blankenfelde-Mahlow (DE); HEINRICH, Friedhelm, 15827 Blankenfelde-Mahlow (DE); KSIANZOU, Viachaslau, 15827 Blankenfelde-Mahlow (DE); STEGLICH, Patrick, 15827 Blankenfelde-Mahlow (DE); BLONDEAU, Jean, 15827 Blankenfelde-Mahlow (DE); VILLRINGER, Claus, 15827 Blankenfelde-Mahlow (DE)
(74) Vertreter: Maikowski & Ninnemann Patentanwälte Partnerschaft mbB

(56) Entgegenhaltungen:
- WO-A1-2011/120688
- DE-A1-102008 002 730
- US-A- 5 068 679
- Beiwen Li ET AL: "High-speed 3D shape measurement with fiber interference High-speed 3D shape measurement with fiber interference", Mechanical Engineering Conference Presentations, Papers, and Proceedings. Paper 70, 31. August 2014 (2014-08-31), XP055282567, Gefunden im Internet: URL:http://lib.dr.iastate.edu/cgi/viewcont ent.cgi?article=1061&context=me_conf [gefunden am 2016-06-22]
- Timothy L Pennington ET AL: "Miniaturized 3-D surface proÿlometer using a ÿber optic coupler", Optics & Laser Technology, 10. Juli 2001 (2001-07-10), Seiten 313-320, XP055282562, Gefunden im Internet: URL:http://ac.els-cdn.com/S003039920100023 8/1-s2.0-S0030399201000238-main.pdf?_tid=f 4f724e2-384b-11e6-8dd2-00000aacb35f&acdnat =1466581136_e03ec401160aec960ac794e9cbfabf ed [gefunden am 2016-06-22]
- ZBIGNIEW KORCZEWSKI: "Analysing the potential for application of the phase shift method in endoscopic examination of marine engines", POLISH MARITIME RESEARCH, Bd. 20, Nr. 1, 12. April 2013 (2013-04-12) , XP055282614, ISSN: 1233-2585, DOI: 10.2478/pomr-2013-0003

## Beschreibung

### Beschreibung

Die Erfindung betrifft einen Messkopf einer endoskopischen Vorrichtung und ein Verfahren zur Inspektion und Messung eines Objektes unter Verwendung eines Endoskops mit einem solchen Messkopf.

Es ist bekannt, für technische und medizinische optische Inspektionen Endoskope einzusetzen. Dabei ist es erforderlich, in Abhängigkeit von der gewünschten Auflösung und Schärfentiefe unterschiedliche Objektive in den Endoskopen zu verwenden.

Die WO 2011/120688 A1 beschreibt eine Blendenvorrichtung, insbesondere für die Fluoreszenzendoskopie. Die Blendenvorrichtung umfasst einen ersten, radial inneren Bereich, der zumindest für einen vorbestimmten Spektralbereich lichtdurchlässig ist. Ferner umfasst sie einen zweiten Bereich, der den ersten Bereich radial außen umgibt und der für einen oder mehrere spektrale Abschnitte aus dem genannten vorbestimmten Spektralbereich eine gegenüber dem Rest des vorbestimmten Spektralbereichs erhöhte Transmission aufweist. Der zweite Bereich wird radial außen von einem dritten Bereich umgeben, der für den gesamten vorbestimmten Spektralbereich lichtundurchlässig oder im Wesentlichen lichtundurchlässig ist.

Der vorliegenden Erfindung liegt die Aufgabe zugrunde, einen Messkopf einer endoskopischen Vorrichtung zur Verfügung zu stellen, der bei unterschiedlichen Messaufgaben und Anforderungen an die Auflösung einsetzbar ist. Des Weiteren soll ein Verfahren zur Inspektion und Messung eines Objektes bereitgestellt werden, das unter Verwendung eines solchen Messkopfes unterschiedliche Messaufgaben realisieren kann.

Diese Aufgabe wird erfindungsgemäß durch einen Messkopf einer endoskopischen Vorrichtung mit den Merkmalen des Anspruchs 1 und ein Verfahren zur Inspektion und Messung eines Objektes mit den Merkmalen des Anspruchs 12 gelöst. Ausgestaltungen der Erfindung sind in den abhängigen Ansprüchen angegeben.

Danach sieht die erfindungsgemäße Lösung einen Messkopf einer endoskopischen Vorrichtung vor, der eine Projektionsoptik zum Beleuchten eines zu untersuchenden Objektes mit Licht und eine Messoptik zum Erfassen des vom zu untersuchenden Objekt reflektierten oder gestreuten Lichts aufweist. Erfindungsgemäß weist die Messoptik eine Aperturblende auf, deren Apertur einstellbar ist.

Durch die Einstellbarkeit der Apertur ist es möglich, bei unveränderten Objektiven von Projektionsoptik und Messoptik unterschiedliche Messaufgaben zu realisieren. So ist es möglich, durch Einstellung einer niedrigen Apertur der Aperturblende größere Bildfelder in einer größeren Schärfentiefe zu erreichen, so dass eine Inspektionsfunktion bereitgestellt werden kann, bei der ein erster Überblick über die durchzuführende optische Inspektion erfolgt. Durch Einstellung einer höheren Apertur wird dagegen bei geringerer Schärfentiefe die Auflösung der Abbildung erhöht, so dass eine Messfunktion realisiert werden kann. Beispielsweise kann vorgesehen sein, sich zunächst unter Verwendung einer niedrigen Apertur einen Überblick über eine durchzuführende Messung zu verschaffen und anschließend unter Einstellung einer hohen Apertur mit hohem Auflösungsvermögen in einem identifizierten Bereich die Messung durchzuführen.

Die Erfindung stellt einen flexibel einsetzbaren Messkopf bereit, der ohne die Notwendigkeit, unterschiedliche Objektive einzusetzen, für unterschiedliche Messaufgaben einsetzbar ist.

Gemäß einer Ausgestaltung der Erfindung ist vorgesehen, dass die Aperturblende durch einen Farbfilter gebildet ist, wobei die Apertur des Farbfilters von der Lichtwellenlänge abhängt. Zur Realisierung eines solchen Farbfilters kann vorgesehen sein, dass der Farbfilter mindestens zwei konzentrische Zonen mit unterschiedlichen Durchlassspektren für Licht aufweist. Beispielsweise ist eine innere konzentrische Zone für Weißlicht durchlässig, während eine um die innere Zone angeordnete äußere konzentrische Zone nur für Licht einer bestimmten Wellenlänge, zum Beispiel blaues Licht, oder für Licht eines Wellenlängenbereiches, durchlässig ist. Die Apertur der Aperturblende ist dabei schrittweise einstellbar. Bei mehr als zwei konzentrischen Zonen kann eine schrittweise Einstellung dabei auch in mehr als zwei Zuständen erfolgen.

Es wird darauf hingewiesen, dass die Aperturblende in der Messoptik an verschiedenen Stellen angeordnet sein kann. Beispielhafte Orte der Anordnung der Aperturblende in der Messoptik sind der Ort der Eintrittspupille, der Ort der Austrittspupille und der Ort einer Öffnungsblende des Objektivs.

Einhergehend mit der Einstellbarkeit der Apertur des Farbfilters in Abhängigkeit von der Lichtwellenlänge ist es erforderlich, dass die endoskopische Vorrichtung, in der der erfindungsgemäße Messkopf enthalten ist, dazu vorgesehen und geeignet ist, Licht unterschiedlicher Wellenlängen für die Messoptik bereitzustellen. Hierzu kann beispielsweise vorgesehen sein, dass in Lichtwellenleiter, die Licht zur Messoptik führen, wahlweise Licht unterschiedlicher Spektren einkoppelbar ist, beispielsweise blaues Licht oder rotes Licht. In einem anderen Beispiel erfolgt die Beleuchtung für die Inspektionsfunktion mit Weißlicht durch Lichtleitfasern im Messkopf, während für die Messfunktion Licht einer bestimmten Wellenlänge oder eines bestimmten Wellenlängenbereichs, beispielsweise rotes Licht, verwendet wird. Dabei kann vorgesehen sein, dass das Licht für die Messfunktion durch eine andere Beleuchtungsoptik bereitgestellt wird, d.h. für die Inspektionsfunktion und für die Messfunktion unterschiedliche Beleuchtungsoptiken eingesetzt werden.

In einer weiteren Ausgestaltung der Erfindung ist vorgesehen, dass der Farbfilter kontinuierlich arbeitet, so dass durch einen Filtergradienten eine kontinuierliche Apertureinstellung erfolgen kann.

Die Strahlengänge in der Messoptik verlaufen für die unterschiedlichen, in Abhängigkeit von der Lichtwellenlänge vorliegenden Aperturen des Farbfilters durch ein Loch, einen Ring oder eine andere Öffnung im Farbfilter.

Die für die Projektionsoptik und die Messoptik verwendeten Objektive können in an sich bekannter Weise aus klassischen Linsenelementen, Asphären und/oder diffraktiven Elementen bestehen.

Gemäß einem alternativen Ausführungsbeispiel der Erfindung ist vorgesehen, dass die Aperturblende durch einen Polarisationsfilter gebildet ist, wobei die Apertur des Polarisationsfilters von der Polarisation des Lichtes abhängt. Hierzu kann beispielsweise vorgesehen sein, dass der Polarisationsfilter mindestens zwei konzentrische Zonen mit unterschiedlichen Polarisationsrichtungen aufweist. Diese Ausgestaltung der Erfindung beruht somit auf dem Gedanken, unterschiedliche numerische Aperturen über einen Polarisationsfilter bereitzustellen, der in unterschiedlichen Bereichen für unterschiedliche Polarisationen durchlässig ist. Beispielsweise kann vorgesehen sein, dass das Zentrum des Filters für alle Polarisationsrichtungen durchlässig ist, während eine um das Zentrum herum angeordnete konzentrische Zone nur für Licht einer Polarisationsrichtung durchlässig ist. Eine alternative Ausgestaltung kann vorsehen, dass das Zentrum nur für Licht einer ersten Polarisationsrichtung durchlässig ist, während eine um das Zentrum herum angeordnete konzentrische Zone nur für Licht einer zweiten Polarisationsrichtung durchlässig ist.

Diese Ausgestaltung der Erfindung sieht vor, unterschiedliche Aperturen durch eine Trennung der Strahlengänge durch Filterzonen unterschiedlicher Polarisationsrichtungen herbeizuführen. Die unterschiedlichen Aperturen können wiederum durch ein Loch, einen Ring oder eine andere Öffnung im Polarisationsfilter realisiert sein.

Auch bei der Einstellbarkeit der Apertur über die Polarisation des Lichtes ist es erforderlich, dass die endoskopische Vorrichtung, in der der erfindungsgemäße Messkopf eingesetzt ist, dazu vorgesehen und ausgebildet ist, Licht unterschiedlicher Polarisationsrichtungen bereitzustellen. Hierzu kann beispielsweise vorgesehen sein, dass in einen Lichtwellenleiter, der Licht der Messoptik zuführt, Licht unterschiedlicher Polarisationsrichtungen einkoppelbar ist.

Gemäß der Erfindung ist vorgesehen, die Aperturblende im Hinblick auf die Apertur einstellbar zu gestalten. Hierzu kann in weiteren Ausführungsbeispielen vorgesehen sein, dass die Aperturblende mechanisch einstellbar ist, z.B. motorische oder handgesteuerte Verstellelemente aufweist, oder anderweilige schaltbare Elemente, die eine Einstellbarkeit der Apertur ermöglichen, aufweist.

Erfindungsgemäß ist ferner vorgesehen, dass die Projektionsoptik Mittel umfasst, oder der Projektionsoptik Mittel des Messkopfes zugeordnet sind, die ein Muster wie z.B. ein ein- oder zweidimensionales Gitter auf das zu untersuchende Objekt projizieren. Das auf das zu untersuchende Objekt projizierte Muster wird anschließend durch die Messoptik erfasst und auf ein Sensorsystem abgebildet, das das Muster im Hinblick auf dreidimensionale (3D)-lnformationen auswerten kann. Auf diese Weise ist eine 3D-Vermessung von Objekten oder Defekten im Rahmen einer hochauflösenden Messung mit hoher Apertur möglich.

Solche 3D-Vermessungen können mithilfe an sich bekannter Triangulationsverfahren erfolgen. Dabei werden durch die Topologie des zu vermessenden Objektes Verzerrungen der Muster auf dem zu untersuchenden Objekt im Hinblick auf die durch diese Verzerrungen bereitgestellten 3D-Informationen ausgewertet. Gemäß dieser Ausgestaltung der Erfindung wird somit eine Messoptik mit variabler Apertur mit einer Projektionsoptik kombiniert, die eine Strukturprojektion auf das zu untersuchende Objekt vornimmt, so dass eine 3D-Vermessung von Objekten oder Strukturen insbesondere durch Triangulationsverfahren ermöglicht wird. Für hochauflösende Messungen im µm-Bereich wird in der Messoptik eine hohe Apertur und für eine Übersichtsinspektion eine niedrige Apertur eingestellt.

Dabei kann vorgesehen sein, dass für die Inspektionsfunktion andere Beleuchtungsmittel vorgesehen sind als für die Messfunktion. Weiter kann vorgesehen sein, dass ein Gitter im Falle der Messfunktion auf das Objekt projiziert wird, das im Falle der Inspektionsfunktion nicht oder zusätzlich abgebildet werden kann.

Diese Ausgestaltung ermöglicht es, bei der technischen und medizinischen Endoskopie neben der allgemeinen Inspektion durch bildgebende Verfahren auch 3D-Messungen der Dimensionen ausgewählter Objekte durchzuführen, wobei insbesondere Höhe, Breite und Tiefe technischer Defekte oder medizinischer Objekte gemessen werden. Durch hochpräzise berührungslose 3D-Messverfahren können beispielsweise technische Wartungsinspektionen von Turbinen und Fahrzeugen wesentliche effizienter gestaltet werden. Mittels der Inspektionsfunktion bei geringer Apertur werden die Defekte grob lokalisiert, um sie danach in der Messfunktion des gleichen Objektives mit hoher Apertur zu vermessen. Wie erläutert, basiert die Auswertung auf einer Triangulationsauswertung eines auf das zu untersuchende Objekt projizierten Musters, bei dem es sich um ein eindimensionales oder zweidimensionales Liniensystem, beispielsweise ein Gitter, handeln kann.

Der gemäß dieser Ausgestaltung der Erfindung vorgesehene Messkopf ist dabei geeignet, auch Strukturen im µm-Bereich dreidimensional zu vermessen und dabei eine Optik mit hohem Auflösungsvermögen und entsprechend großer Apertur zu verwenden. Dies ist bei bisher in der Endoskopie eingesetzten Verfahren zur 3D-Messung, insbesondere dem so genannten Phasenverfahren, wie es aus der US 2010/0296104 A1 bekannt ist, nicht möglich.

Gemäß einer ersten erfindungsgemäßen Alternative weisen die genannten Mittel, die ein Muster auf das zu untersuchende Objekt projizieren, ein Transmissionsgitter auf. Dieses kann mehrere unterschiedliche Gitterkonstanten in unterschiedlichen Gitterbereichen aufweisen. Durch die unterschiedlichen Gitterkonstanten können wahlweise unterschiedliche Frequenzen aufgelöst werden, in Abhängigkeit davon, welcher Gitterbereich des Transmissionsgitters auf das Objekt abgebildet wird. Insofern wird das Gitter durch die Bewegung des Messkopfes auf das Objekt projiziert.

Eine zweite erfindungsgemäße Alternative sieht vor, dass die Mittel, die ein Muster auf das zu untersuchende Objekt projizieren, mindestens zwei Monomode-Lichtwellenleiter umfassen, deren kohärentes Licht unter Bildung eines Gitters miteinander interferiert, wobei der Abstand der Lichtwellenleiter zur Einstellung der Gitterkonstanten einstellbar ist. Alternativ oder ergänzend kann die Gitterkonstante auch durch Änderung der Wellenlänge des verwendeten Lichts bei festem Abstand der Lichtwellenleiter zueinander eingestellt werden. Diese Ausgestaltung der Erfindung ermöglicht es durch Ausnutzung der physikalischen Vorgänge der Beugung und Interferenz in einfacher Weise, ein Gitter zu erzeugen, dessen Gitterkonstante einstellbar ist.

Wird die Phasenbeziehung des Lichts der beiden Monomodefasern zueinander kontrolliert eingestellt, so kann das erzeugte Gitter ohne mechanisch bewegte Teile senkrecht zu den Linien bewegt werden, was scannende Funktionen ermöglicht.

Eine weitere Ausgestaltung der Erfindung sieht vor, dass die optischen Achsen von Projektionsoptik und Messoptik unter einem Winkel verlaufen, d.h. die Projektionsoptik und die Messoptik zueinander geneigt sind. Dies ist insbesondere sinnvoll bei Triangulationsmessungen unter Auswertung eines auf das zu untersuchende Objekt projizierten Musters, da es für Triangulationsmessungen erforderlich ist, eine schräge Anordnung bzw. einen Winkel zwischen der Projektionsoptik und der Messoptik bzw. den jeweiligen optischen Achsen bereitzustellen.

Eine Ausgestaltung der Erfindung sieht vor, dass die Projektionsoptik derart ausgebildet ist, dass sie eine dezentrierte Abbildung bereitstellt. Hierzu kann beispielsweise vorgesehen sein, dass die Beleuchtungsfaser, die Licht in die Projektionsoptik einkoppelt, nicht auf der optischen Achse der Projektionsoptik Licht einkoppelt, sondern dezentriert, das heißt, parallel zu dieser optischen Achse. Hierdurch wird ein gleichmäßigeres Ausleuchten im Überlappungsbereich der Mess- und Projektionsoptik ermöglicht. Dabei kann vorgesehen sein, dass auch die Mittel, die ein Muster auf das zu untersuchende Objekt projizieren (beispielsweise ein Transmissionsgitter) dezentriert bezogen auf die optische Achse der Projektionsoptik angeordnet ist. Hierdurch wird eine zusätzliche Schräge bzw. ein größerer Triangulationswinkel für eine Triangulationsmessung ermöglicht.

Gemäß einer Ausgestaltung der Erfindung ist vorgesehen, dass der Messkopf des Weiteren einen Sensor aufweist, der dazu vorgesehen und ausgebildet ist, das der Messoptik erzeugte Bild zu erfassen und ggf. weiter auszuwerten. Der Sensor erzeugt dabei ein Monitorbild und erlaubt eine digitale Bildverarbeitung. Es kann jedoch ebenfalls vorgesehen sein, dass das durch die Projektionsoptik bereitgestellte Bild z.B. mittels einer Bildleitfaser, die jedem Pixelpunkt eine einzelne Lichtleitfaser zuordnet, aus dem Messkopf und dem Endoskop herausgeführt wird und der Sensor zum Erfassen und Auswerten des von der Messoptik erfassten Lichtes außerhalb des Messkopfes und/oder des Endoskops angeordnet ist. Hierdurch wird eine zusätzliche Flexibilität in der Anordnung des Sensors bereitgestellt.

Es wird darauf hingewiesen, dass die Projektionsoptik derart ausgebildet sein kann, dass sie bei allen Realisierungen der Aperturblende nur eine Optik aufweist, die Licht bereitstellt und auf das Objekt abbildet. Dabei kann wie erläutert vorgesehen sein, dass Licht unterschiedlicher Wellenlängen oder Polarisationsebenen in diese eine Optik eingekoppelt wird, um unterschiedliche Aperturen zu realisieren.

Es liegt jedoch ebenfalls im Rahmen der vorliegenden Erfindung, dass die Projektionsoptik zwei Optiken aufweist, die jeweils gesondert für die Projektionsfunktion und die Messfunktion eingesetzt werden. So sieht eine Ausgestaltung der Erfindung vor, dass die Projektionsoptik eine erste Beleuchtungsoptik und eine zweite Beleuchtungsoptik aufweist, wobei die erste Beleuchtungsoptik dazu ausgebildet und vorgesehen ist, Licht bereitzustellen, wenn die Aperturblende eine erste Apertur realisiert, und die zweite Beleuchtungsoptik dazu ausgebildet und vorgesehen sind, Licht bereitzustellen, wenn die Aperturblende eine zweite Apertur realisiert. Die erste Beleuchtungsoptik umfasst beispielsweise Lichtwellenleiter und eine Abbildungsoptik und ggf. zusätzlich einen Kondensor und ein Gitter. Sie wird beispielsweise für die Messfunktion verwendet. Die zweite Beleuchtungsoptik umfasst beispielsweise ein Lichtleitfaserbündel. Sie wird beispielsweise für die Inspektionsfunktion verwendet.

Gemäß einem weiteren Aspekt der Erfindung, der von der Ausgestaltung der von der Projektionsoptik umfassten oder dieser zugeordneten Mittel, die zum Projizieren eines Musters auf das zu untersuchende Objekt vorgesehen sind, unabhängig ist, ist dementsprechend ein Messkopf einer endoskopischen Vorrichtung vorgeschlagen, der eine Projektionsoptik aufweist, die dazu vorgesehen und ausgebildet ist, ein zu untersuchendes Objekt mit Licht zu beleuchten, sowie eine Messoptik aufweist, die dazu vorgesehen und ausgebildet ist, vom zu untersuchenden Objekt reflektiertes oder gestreutes Licht zu erfassen. Die Messoptik weist auch hier eine Aperturblende auf, deren Apertur einstellbar ist. Ferner ist vorgesehen, dass die Projektionsoptik eine erste Beleuchtungsoptik und eine zweite Beleuchtungsoptik aufweist, wobei die erste Beleuchtungsoptik dazu ausgebildet und vorgesehen ist, Licht bereitzustellen, so dass die Aperturblende eine erste Apertur realisiert, und die zweite Beleuchtungsoptik dazu ausgebildet und vorgesehen sind, Licht bereitzustellen, so dass die Aperturblende eine zweite Apertur realisiert.

Die Erfindung stellt auch ein Verfahren zur Inspektion und Messung eines Objektes bereit. Dieses Verfahren erfolgt unter Verwendung eines Endoskops mit einem Messkopf, der aufweist:
- eine Projektionsoptik, die dazu vorgesehen und ausgebildet ist, ein zu untersuchendes Objekt mit Licht zu beleuchten, und
- eine Messoptik, die dazu vorgesehen und ausgebildet ist, vom zu untersuchenden Objekt reflektiertes oder gestreutes Licht zu erfassen.
Die Projektionsoptik umfasst Mittel oder der Projektionsoptik sind Mittel zugeordnet, die ein Muster auf das zu untersuchende Objekt projizieren. Die Messoptik weist ferner eine Aperturblende auf, deren Apertur einstellbar ist. Im Rahmen eines erfindungsgemäßen Verfahrens wird eine kleinere Apertur zur Realisierung einer Inspektionsmessung mit geringer Auflösung aber größerer Schärfentiefe und eine größere Apertur zur Realisierung einer Messfunktion mit höherer Auflösung, jedoch geringere Schärfentiefe eingestellt, wobei im Fall der Messfunktion ein Muster auf das Objekt projiziert wird, das im Fall der Inspektionsfunktion nicht oder zusätzlich abgebildet wird.

Eine Ausgestaltung des Verfahrens sieht vor, dass bei Verwendung der größeren Apertur Licht einer ersten Beleuchtungsoptik zur Objektbeleuchtung und bei Verwendung der kleineren Apertur Licht einer zweiten Beleuchtungsoptik zur Objektbeleuchtung verwendet wird. Dabei kann vorgesehen sein, dass bei Verwendung der kleineren Apertur Lichtleitfaserbündel zur Objektbeleuchtung eingesetzt werden.

Gemäß einer weiteren Ausgestaltung wird bei Verwendung der größeren Apertur ein Muster, beispielsweise ein Gitter auf das zu untersuchende Objekt projiziert.

Es liegt dabei natürlich im Rahmen der Erfindung, dass das Verfahren unter Verwendung eines Endoskops mit einem erfindungsgemäßen Messkopf durchgeführt wird.

Die Erfindung wird nachfolgend unter Bezugnahme auf die Figuren der Zeichnung anhand mehrerer Ausführungsbeispiele näher erläutert. Es zeigen:
- Figur 1: schematisch ein erstes Ausführungsbeispiel eines Messkopfes mit einer Projektionsoptik, einer Messoptik und einer einstellbaren Aperturblende;
- Figur 2A: ein erstes Ausführungsbeispiel eines im Hinblick auf die Apertur einstellbaren Farbfilters;
- Figur 2B: ein zweites Ausführungsbeispiel eines im Hinblick auf die Apertur einstellbaren Farbfilters;
- Figur 3: eine weitere Darstellung des Farbfilters der Figur 2A unter Darstellung der transmittierten Wellenlängen;
- Figur 4: ein Ausführungsbeispiel einer Messoptik unter Verwendung von Licht einer ersten Wellenlänge und mit hoher Apertur;
- Figur 5: die Messoptik der Figur 4 unter Verwendung einer zweiten Wellenlänge und mit geringer Apertur;
- Figur 6: die Simulation der Abbildung eines Spaltes, wenn die Messoptik eine Inspektionsfunktion mit geringer Apertur realisiert;
- Figur 7: die Simulation der Abbildung eines Spaltes, wenn die Messoptik eine Messfunktion mit einer hohen Apertur realisiert;
- Figur 8A: ein erstes Ausführungsbeispiel eines im Hinblick auf die Apertur einstellbaren Polarisationsfilters;
- Figur 8B: ein zweites Ausführungsbeispiel eines im Hinblick auf die Apertur einstellbaren Polarisationsfilters;
- Figur 9A: schematisch ein zweites Ausführungsbeispiel eines Messkopfes, der eine Projektionsoptik, eine Messoptik und eine einstellbare Aperturblende aufweist;
- Figur 9B: eine Abwandlung des Ausführungsbeispiels der Figur 9A, bei der zusätzlich eine allgemeine Beleuchtung des Objektes vorgesehen ist;
- Figur 10: einen Messkopf entsprechend dem Messkopf der Figur 9A, wobei die Strahlengänge bei einer dezentrierten Einkopplung von Licht in die Projektionsoptik dargestellt sind;
- Figur 11: ein Ausführungsbeispiel eines Transmissionsfilters, der bei dem Messkopf der Figur 9A, 9B oder der Figur 10 eingesetzt wird;
- Figur 12: schematisch ein drittes Ausführungsbeispiel eines Messkopfes mit einer Projektionsoptik und einer Messoptik mit einstellbarer Apertur, wobei die Projektionsoptik durch eine Mehrzahl von Lichtwellenleitern realisiert ist;
- Figur 13: eine schematische Darstellung des Funktionsprinzips der Verwendung von zwei Monomode-Lichtwellenleitern zur Erzeugung eines Gitters;
- Figur 14: ein viertes Ausführungsbeispiel eines Messkopfes, der eine Projektionsoptik, eine Messoptik, eine einstellbare Aperturblende und eine allgemeine Objektbeleuchtung aufweist; und
- Figur 15: die Simulation einer Abbildung eines durch einen Defekt verformten, auf das zu untersuchende Objekt abgebildeten Gitters.

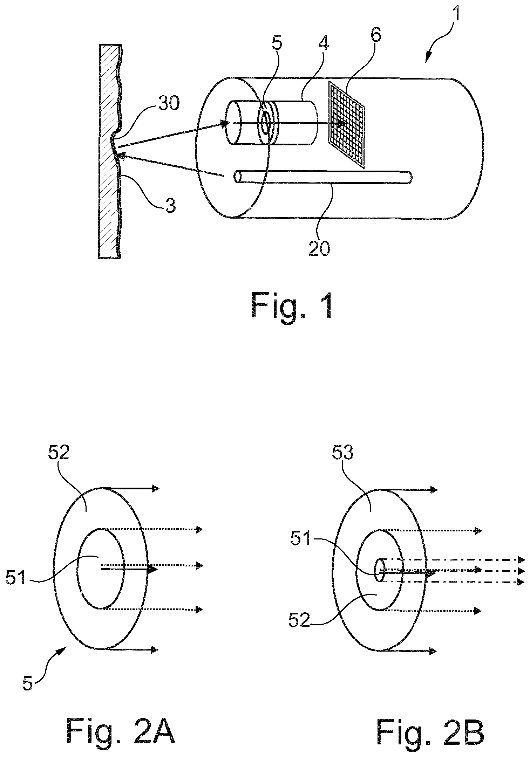

Die Figur 1 zeigt ein erstes Ausführungsbeispiel eines Messkopfes 1 einer endoskopischen Vorrichtung, die eine Messoptik mit einstellbarer Apertur aufweist. Der Messkopf 1 umfasst eine Projektionsoptik 20, eine Messoptik 4 mit einer integrierten Aperturblende 5 und einen Sensor 6.

Die Projektionsoptik 20 ist im dargestellten Ausführungsbeispiel durch einen einzigen Lichtwellenleiter gebildet, der Licht auf ein zu untersuchendes Objekt 3 ausstrahlt, das beispielsweise einen Defekt 30 aufweist. Das von der Projektionsoptik 20 ausgestrahlte Licht wird an dem zu untersuchenden Objekt 3 reflektiert und gestreut und von der Messoptik 4 erfasst. Die Messoptik 4 bildet die zu untersuchende Oberfläche des zu untersuchenden Objektes 3 auf den Sensor 6 ab. Der Sensor erzeugt ein Monitorbild, das eine digitale Bildverarbeitung erlaubt und weiterverarbeitet werden kann.

Es wird darauf hingewiesen, dass die Projektionsoptik 20 nur im einfachsten Fall aus einem einzelnen Lichtwellenleiter besteht. Wie noch ausgeführt werden wird, kann die Projektionsoptik auch aus einem Linsensystem z.B. mit klassischen Linsenelementen, Asphären und/oder diffraktiven Elementen bestehen, oder aus mehreren Lichtwellenleitern.

Die in die Messoptik 4 integrierte Aperturblende 5 ist im Hinblick auf ihre Apertur einstellbar. Die Einstellbarkeit erfolgt beispielsweise dadurch, dass die Aperturblende durch einen Farbfilter oder durch einen Polarisationsfilter gebildet ist, wobei die Apertur des Filters von der Lichtwellenlänge bzw. der Polarisation des Lichts abhängt.

Die Figuren 2A, 2B zeigen zwei Ausführungsbeispiele für Aperturblenden, die durch einen Farbfilter gebildet sind. Bei der Figur 2A ist eine zentrale Filterzone 51 für sichtbares Licht sämtlicher Wellenlängen durchlässig. In einem konzentrisch dazu angeordneten Außenbereich 52 ist der Farbfilter dagegen nur für das Spektrum einer der Farbgruppen des Sensors durchlässig, beispielsweise für blaues Licht. Wenn beispielsweise blaues Licht eingekoppelt wird, so sind beide Bereiche 51, 52 für das blaue Licht durchlässig, so dass eine hohe Apertur realisiert ist. Wird dagegen rotes Licht eingekoppelt, so wird dieses nur durch die zentrale Filterzone 51 durchgelassen, so dass eine kleine Apertur realisiert ist. In Abhängigkeit von der Wellenlänge des verwendeten Lichts können somit zwei unterschiedliche Aperturen automatisch bereitgestellt werden.

Die Figur 2B zeigt ein Ausführungsbeispiel für einen Farbfilter, der drei unterschiedliche numerische Aperturen realisieren kann, in Abhängigkeit davon, ob Licht nur durch die zentrale Filterzone 51 oder zusätzlich durch die weiteren konzentrischen Zonen 52, 53 treten kann. Beispielsweise ist die zentrale Filterzone 51 für Licht sämtlicher Wellenlängen durchlässig. Die sich daran anschließende konzentrische Filterzone 52 ist für Licht einer ersten Wellenlänge oder eines ersten Wellenlängenbereichs und die zweite konzentrische Filterzone 53 für Licht einer zweiten Wellenlänge oder eines zweiten Wellenlängenbereichs durchlässig.

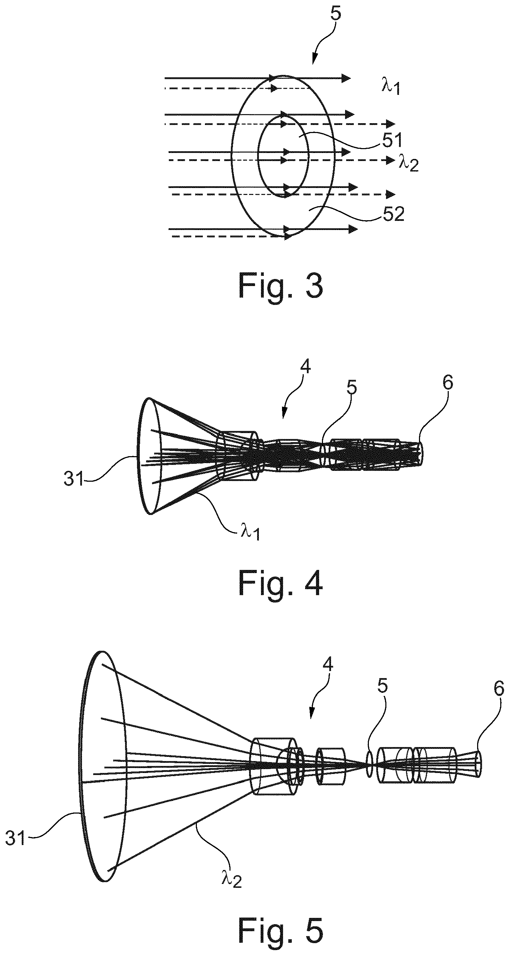

Die Figuren 3 bis 5 verdeutlichen weitergehend die Funktionsweise eines als Aperturblende wirkenden Farbfilters gemäß der Figur 2A. Bei dem Farbfilter 5 der Figur 3 ist die zentrale Filterzonen 51 für weißes Licht (und damit für blaues Licht der Wellenlänge λ₁ oder des Wellenlängenbereiches Δλ₁ und für rotes Licht der Wellenlänge λ₂ oder des Wellenlängenbereiches Δλ₂) durchlässig. Die sich konzentrisch daran anschließende Filterzone 52 ist nur für blaues Licht der Wellenlänge λ₁ oder des Wellenlängenbereiches Δλ₁ durchlässig.

Sofern über die Projektionsoptik 20 blaues Licht der Wellenlänge λ₁ eingekoppelt wird, ergibt sich eine hohe Aperturblende, da beide Zonen 51, 52 das blaue Licht der Wellenlänge λ₁ transmittieren. Die hohe Apertur korrespondiert mit einem hohen Auflösungsvermögen und einer geringen Schärfentiefe. Wenn durch die Projektionsoptik 20 rotes Licht eingekoppelt wird, blockiert der äußere Filterbereich 52 das rote Licht, so dass dieses nur von der zentralen Filterzone 51 durchgelassen wird. Dementsprechend ist nun eine niedrige Apertur realisiert, die mit einer höheren Schärfentiefe und einem geringeren Auflösungsvermögen korrespondiert. Dies ist in den Figuren 4 und 5, die die Messoptik 4 zeigen, für die Wellenlängen λ₁ und λ₂ dargestellt.

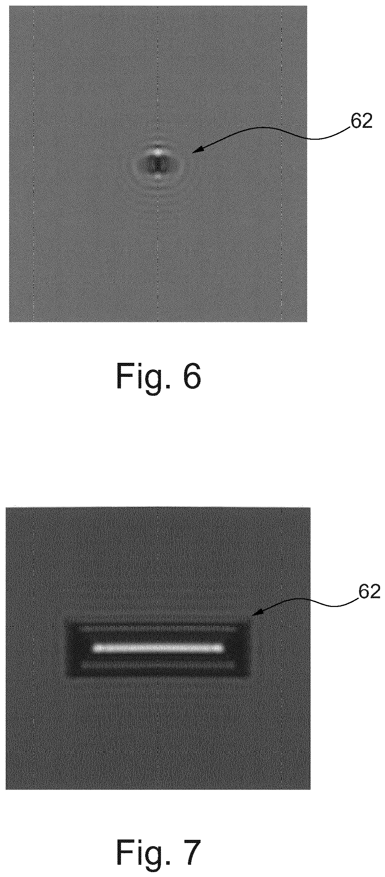

Die Figur 6 zeigt die Abbildung eines 50 µm x 150 µm großen Defektes 62 (z.B. eines Grabens) bei Verwendung einer kleinen Apertur der Aperturblende 5. Beispielsweise liegt die numerische Apertur bei 0,02. Der Defekt 62 ist erkennbar, aufgrund der kleinen Apertur und der damit korrespondierenden geringen Auflösung erkennbar, aber nicht aufgelöst. In der Figur 7 ist derselbe Defekt 62 unter Verwendung der großen Apertur der Aperturblende 5 dargestellt. Der Defekt 62 ist aufgrund des höheren Auflösungsvermögens bei der großen Apertur nun aufgelöst und kann vermessen werden.

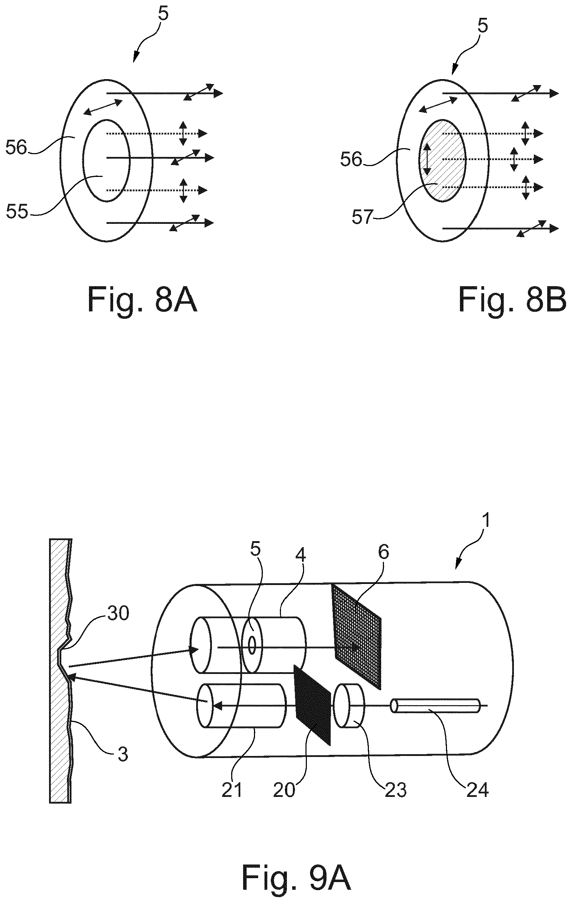

Die Figuren 8A, 8B zeigen zwei Ausführungsbeispiele für Aperturblenden entsprechend der Aperturblende 5 der Figur 1, die durch Polarisationsfilter ausgebildet sind.

Die Figur 8A zeigt ein erstes Ausführungsbeispiel eines Polarisationsfilters mit zwei numerischen Aperturen. Eine zentrale Filterzone 55 ist für alle Polarisationsrichtungen durchlässig, während der sich darum konzentrisch erstreckende Ring 56 nur für in x-Richtung (senkrecht zur Blattebene) polarisiertes Licht transparent ist.

Wenn über die Projektionsoptik 20 in x-Richtung polarisiertes Licht abgestrahlt und nach Reflexion oder Streuung an dem zu untersuchenden Objekt 3 durch die Projektionsoptik 4 erfasst wird, so ist der Polarisationsfilter 5 in beiden Zonen 55, 56 durchlässig, da das Zentrum 55 für beide Polarisationsrichtungen und der äußere Ring 56 für die Polarisation in x-Richtung durchlässig ist. Wenn dagegen in y-Richtung polarisiertes Licht eingekoppelt wird, ist nur die zentrale Filterzone 55 durchlässig, da die äußere Filterzone 56 in y-Richtung polarisiertes Licht blockiert. Dementsprechend ergibt sich eine hohe numerische Apertur für in x-Richtung polarisiertes Licht und eine niedrige numerische Apertur für in y-Richtung polarisiertes Licht.

Es ergeben sich die identischen Darstellungen wie in den Figuren 4 und 5, wobei die Apertur der Aperturblende 5 anders als in den Figuren 4 und 5 nicht von der Wellenlänge, sondern von der Polarisationsrichtung abhängt. Es ergeben sich jedoch die gleichen Strahlengänge, die auch in den Figuren 4 und 5 abgebildet sind.

Die Figur 8B zeigt ein weiteres Ausführungsbeispiel eines Polarisationsfilters 5. Bei dieser Ausgestaltung ist die zentrale Filterzone 57 nur für den in y-Richtung (parallel zur Blattebene) polarisierten Anteil des Lichtes durchlässig, während die äußere Filterzone 56 nur für den in x-Richtung polarisierten Anteil des Lichts durchlässig ist. Damit ergibt sich, dass der Polarisationsfilter bei in x-Richtung polarisiertem Licht nur in seinem Ringbereich 56 durchlässig und bei in y-Richtung polarisiertem Licht nur in seinem zentralen Bereich 57 lichtdurchlässig ist. Wiederum liegt ein in seiner Apertur einstellbarer Filter vor.

Die Figur 9A zeigt ein weiteres Ausführungsbeispiel eines erfindungsgemäßen Messkopfes. Dieser Messkopf unterscheidet sich von dem Messkopf der Figur 1 insbesondere durch die Ausgestaltung der Projektionsoptik. Diese umfasst einen Lichtwellenleiter 24, der Licht heranführt, einen Kondensor 23, ein Gitter 22 und als eigentliche Projektionsoptik eine Gitterabbildungsoptik 21, die das Gitter 22 auf das zu untersuchende Objekt 3 (das beispielsweise einen Defekt 30 aufweist) abbildet. Das reflektierte Bild wird über in die Optik 4, die wiederum - ebenso wie in Bezug auf die Figur 1 beschrieben - eine variable Aperturblende 5 aufweist, erfasst und auf einen Sensor 6 abgebildet.

Die Messoptik 4 kann somit unterschiedliche Aperturen über eine einstellbare Aperturblende realisieren. Insofern wird auf die Ausführungen zu den Figuren 1 bis 8B verwiesen. Das Messobjektiv 4 ist somit in der Lage, sowohl eine Inspektionsfunktion als auch eine Messfunktion zu realisieren. Die Messoptik 4 bildet die zu untersuchende Oberfläche dabei auf den Sensor 6 ab. Der Sensor 6 erzeugt ein Monitorbild und erlaubt eine digitale Bildverarbeitung. Dabei kann vorgesehen sein, dass das Licht zunächst auf eine Bildleitfaser projiziert und von dieser zu einem außerhalb des Endoskops angeordneten Sensor geleitet wird.

Die Projektionsoptik ist dafür vorgesehen, dass durch das Gitter 22 bereitgestellte Muster auf die Oberfläche des zu untersuchenden Objektes 3 zu projizieren. Das Muster, z.B. ein Gitter, wird dabei durch eine Beleuchtungsoptik bestehend aus dem Kondensor 23 und dem Lichtleiter 24 ausgeleuchtet. Das Objektiv der Messoptik 4 ist so ausgebildet, dass es in der Lage ist, das durch die Topografie verformte Muster auf den Sensor 6 aufgelöst abzubilden. Durch Bildverarbeitung können dann Defekte in der x- und y-Dimension vermessen werden. Durch zusätzliche Anwendung von Triangulationsverfahren kann die Tiefe z am Ort x, y aus der Kenntnis des Einfalls- und Beobachtungswinkels und der Deformation der Linie bestimmt werden. Somit kann eine vollständige Rekonstruktion der Oberfläche einschließlich der Defekte erfolgen.

Bei der Figur 9A ist vorgesehen, dass die Projektionsoptik (die den Lichtwellenleiter 24, den Kondensor 23, das Gitter 22 und die Gitterabbildungsoptik 21 umfasst) Licht sowohl bereitstellt und auf das Objekt 3 abbildet, wenn die Aperturblende 5 eine kleine Apertur und damit eine Inspektionsfunktion realisiert, als auch dann, wenn die Aperturblende 5 eine große Apertur und damit eine Messfunktion realisiert. Dies ist jedoch keineswegs notwendigerweise der Fall.

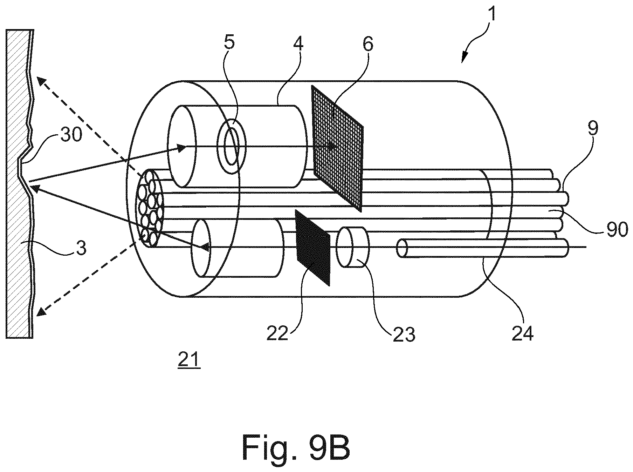

So zeigt die Figur 9B eine Abwandlung des Ausführungsbeispiels der Figur 9A, bei der die Projektionsoptik eine zusätzliche Beleuchtungsoptik 9 in Form eines Lichtleitfaserbündels 90 umfasst. Alternativ könnte die zusätzliche Beleuchtungsoptik 9 z.B. durch eine LED oder eine LED mit Lichtleitfaserbündel realisiert sein. Die zusätzliche Beleuchtungsoptik 9 stellt die Objektbeleuchtung bereit, wenn der Messkopf 1 für eine Inspektionsfunktion eingesetzt wird. Wenn der Messkopf 1 für eine Messfunktion eingesetzt wird, wird dagegen die Projektionsoptik mit Lichtwellenleiter 24, Kondensor 23, Gitter 22 und Gitterabbildungsoptik 21 eingesetzt.

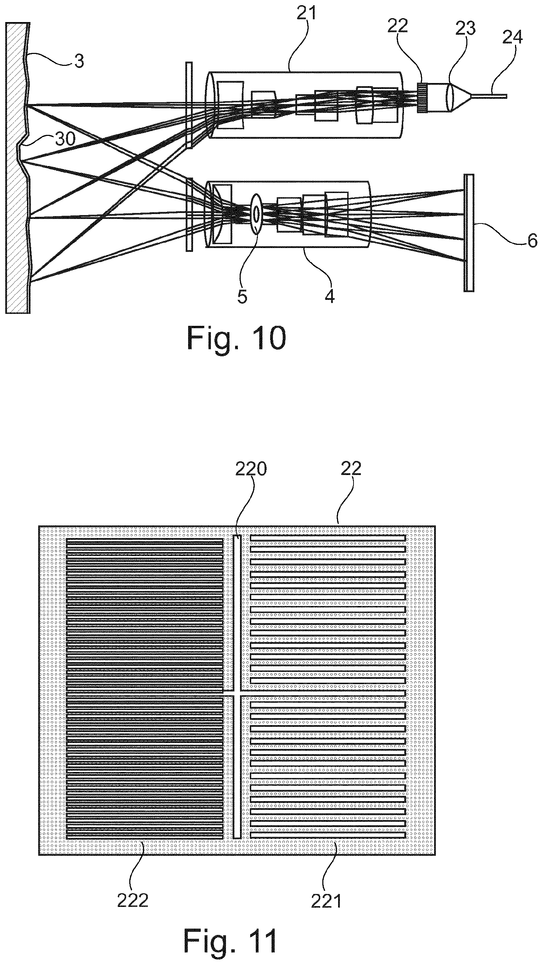

Die Figur 10 zeigt konkreter die Arbeitsweise eines Messsystems gemäß der Figur 9. Dabei ist vorgesehen, dass die Lichteinkopplung von der Lichtleitfaser 24 über den Kondensor 23 und das Gitter 22 in die Gitterabbildungsoptik 21 dezentriert erfolgt, d.h. nicht symmetrisch zur optischen Achse der Gitterabbildungsoptik 21, sondern parallel versetzt zu dieser. Dies ist in der Figur 10 schematisch dargestellt. Hierdurch wird erreicht, dass das durch die Projektionsoptik schräg auf die Oberfläche des zu untersuchenden Objektes 3 projizierte Muster mit einem schrägeren Winkel auffällt und dadurch der Triangulationswinkel, der für eine Triangulationsmessung erforderlich ist, erhöht wird. Eine zusätzliche Dezentrierung der Lichtleitfaser, so dass mindestens eine erste Beugungsordnung ohne Abschattung die Gitterabbildungsoptik durchläuft, ermöglicht es, das Auflösungsvermögen im Messfenster auf dem Objekt zu homogenisieren.

Das durch die Projektionsoptik 21, 22, 23, 24 schräg auf die Oberfläche projizierte Muster wird schräg durch die Messoptik 4 detektiert. Somit erhält man den für eine Triangulation notwendigen Winkel als Summe beider Winkel. Die Winkeleinstellung wird, wie bereits erwähnt, durch die dezentrierte Abbildung der Projektionsoptik erreicht. Sie kann zusätzlich oder alternativ durch zusätzliche optische Bauelemente wie z.B. Spiegel, Prismen, Beugungsgitter und diffraktive Elemente realisiert werden. Auch kann vorgesehen sein, dass die Messoptik 4 und die Projektionsoptik winklig zueinander angeordnet sind, d.h. zueinander geneigt sind und ihre optischen Achsen einen Winkel bilden (nicht gesondert dargestellt).

Eine Einstellung der Apertur durch die Aperturblende 5 erfolgt beispielsweise - wie in Bezug auf die Figuren 1 bis 8B beschrieben - durch Realisierung der Aperturblende durch einen Farbfilter oder durch einen Polarisationsfilter. Auch kann vorgesehen sein, dass die Einstellbarkeit der Aperturblende auf andere Weise erfolgt, etwa mechanisch, beispielsweise durch motorische Verstellelemente, elektrochrome oder thermochrome Elemente. Aperturblenden auf der Basis von Polarisationsfiltern können durch den Einsatz von Flüssigkristallen auch elektrisch schaltbar gestaltet sein. Die in ihrer Apertur einstellbare Aperturblende 5 ist in der Figur 10 ebenfalls schematisch dargestellt. Im Hinblick auf die durch die unterschiedliche Apertur realisierten unterschiedlichen Strahlengänge zur Realisierung zum einen einer Inspektionsfunktion und zum anderen zur Realisierung einer Messfunktion wird wiederum auf die Figuren 4 und 5 verwiesen, die auch für das Ausführungsbeispiel der Figuren 9 und 10 gelten.

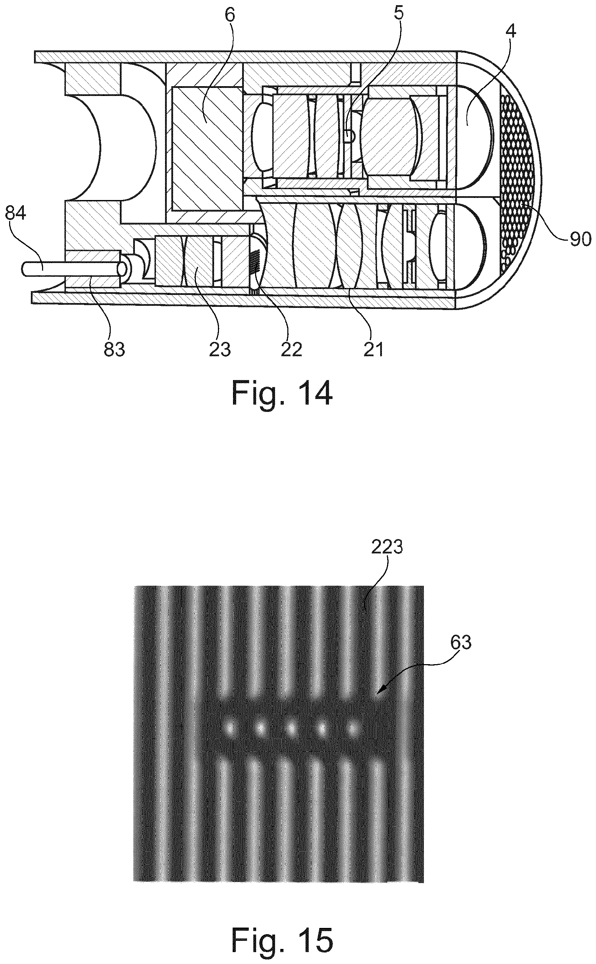

Die Figur 15 zeigt die Simulation der Abbildung eines Gitters auf die Oberfläche eines zu untersuchenden Objektes, wobei das Gitter einen Pitch von 20 µm und eine Spaltbreite von 7µm aufweist. Das Gitter ist auf eine Oberfläche projiziert, die einen Defekt 63 in Form eines Kratzers mit einer Breite von 50 µm und einer Tiefe von 20 µm aufweist. Dementsprechend sind in der Figur 15 die Gitterlinien im Bereich des Defekts 63 verformt. Die Messoptik 4 hat im betrachteten Ausführungsbeispiel eine Apertur von NAₘ=0,1 mit einer Defokussierung von 25 µm und einer Wellenlänge von 633 nm. Im Messmodus (hohe Apertur) kann das Bild mit den deformierten Gitterlinien durch die Projektionsoptik 4 aufgelöst auf den Sensor 6 abgebildet werden, sodass mittels Triangulationsverfahren aus der Verformung der Gitterlinien die dreidimensionale Form des Defektes 63 ermittelt werden kann.

In den Figuren 9A, 9B und 10 ist das Gitter als Transmissionsgitter realisiert, das beispielsweise als Glasplatte ausgebildet ist, auf die z.B. Gitterstrukturen aus Chrom aufgebracht sind. Ein Beispiel eines solchen Transmissionsgitters zeigt die Figur 11. Das Transmissionsgitter 22 bildet dabei unterschiedliche Gitterkonstanten in den Bereichen 221, 222 ab. Zusätzlich kann das Gitter ein zentrales Element, zum Beispiel ein Kreuz 220 umfassen. Die unterschiedlichen Gitterkonstanten können in Abhängigkeit von der eingestellten Auflösung bzw. Apertur eingesetzt werden und dienen der Justage, Bildverarbeitung und Erhöhung der Eindeutigkeit der Rekonstruktion.

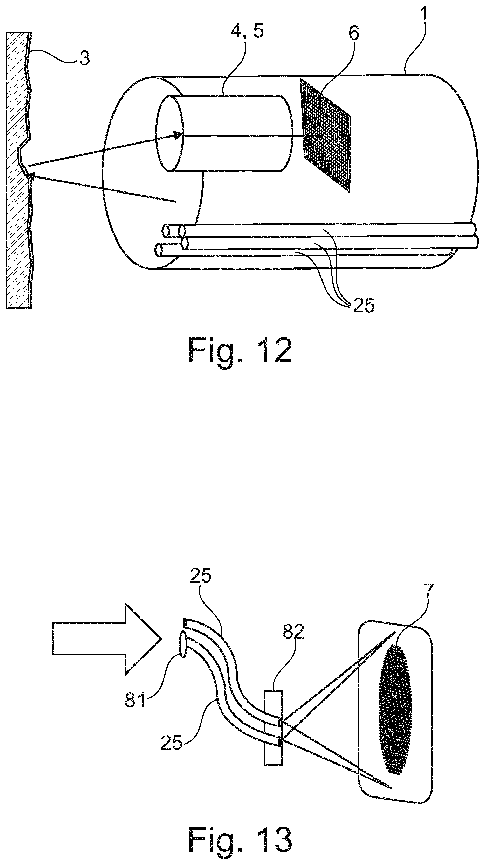

Die Figuren 12 und 13 zeigen ein alternatives Ausführungsbeispiel zur Realisierung eines Gitters. Im Ausführungsbeispiel der Figur 12 ist ein Messkopf 1 dargestellt, bei dem die Projektionsoptik 21, 22, 23, 24 der Figuren 9 und 10 durch eine Mehrzahl von parallel angeordneten Monomode-Lichtwellenleitern 25 ersetzt ist. Diese Monomode-Lichtwellenleiter sind so angeordnet, dass das von ihnen ausgestrahlte Licht miteinander interferiert, wobei ein Gitter entsteht. Dies ist in der Figur 13 schematisch dargestellt. In zwei Monomode-Fasern 25 wird Licht eines Lasers eingekoppelt, wobei die Einkopplung in eine der Monomode-Fasern unter Verwendung eines λ/2-Plättchen 81 zur Abstimmung der Polarisation der Fasern erfolgt. Das λ/2-Plättchen 81 kann entfallen, wenn polarisationserhaltende Monomodefasern eingesetzt werden. In einer Halterung 82 werden die beiden Monomode-Fasern 25 in definiertem Abstand gehalten. Durch Interferenz, die der Interferenz an einem Doppelspalt entspricht, entsteht ein Gitter 7. Der Vorteil dieser Ausgestaltung besteht darin, dass der Gitterabstand entweder durch den Abstand der beiden Monomode-Fasern 25 im Bereich der Halterung 82 oder durch Variation der eingestrahlten Wellenlänge in einfacher Weise eingestellt und somit die Triangulationsmessung verbessert werden kann.

Bei allen Ausführungsbeispielen und damit auch bei dem Ausführungsbeispiel der Figuren 12 und 13 kann vorgesehen sein, dass die Projektionsoptik eine gesonderte Beleuchtungsoptik aufweist (entsprechend der Beleuchtungsoptik 9 der Figur 9B), mittels derer das Objekt 3 bei Realisierung der Inspektionsfunktion beleuchtet wird.

Die Figur 14 zeigt ein weiteres Ausführungsbeispiel eines erfindungsgemäßen Messkopfes. Dieses Ausführungsbeispiel zeigt beispielhaft Linsensysteme, die die Projektionsoptik 21, 22, 23 und die Messoptik 4 realisieren. Eine Halterung 83 ist zum Halten einer Lichtleitfaser 84 vorgesehen, die Beleuchtungslicht für die Gitterabbildung und die Messfunktion zuführt. Es ist gut zu erkennen, dass sowohl das Gitter 22 dezentriert bezogen auf die Abbildungsoptik 21 angeordnet ist als auch die Einkopplung von Licht in die Abbildungsoptik 21 dezentriert erfolgt, d.h. versetzt zur optischen Achse der Abbildungsoptik 21. Der Sensor ist durch eine Sensorbaugruppe 6 realisiert. Wiederum ist eine Aperturblende 5 mit einstellbarer Apertur vorgesehen, die beispielsweise als Farbfilterblende oder als Polarisationsfilterblende ausgebildet ist. Die Beleuchtung für die Inspektionsfunktion erfolgt entsprechend der Figur 9B durch ein Lichtleitfaserbündel 90, das schematisch dargestellt ist.

Die Erfindung beschränkt sich in ihrer Ausgestaltung nicht auf die vorstehend dargestellten Ausführungsbeispiele, die lediglich beispielhaft zu verstehen sind. Insbesondere ist die Erfindung nicht auf bestimmte Ausgestaltungen einer Projektionsoptik, einer Messoptik oder einer Aperturblende beschränkt.

Des Weiteren wird darauf hingewiesen, dass die Merkmale der einzelnen beschriebenen Ausführungsbeispiele der Erfindung in verschiedenen Kombinationen miteinander kombiniert werden können. Sofern Bereiche definiert sind, so umfassen diese sämtliche Werte innerhalb dieser Bereiche sowie sämtliche Teilbereiche, die in einen Bereich fallen.

## Patentansprüche

1. Messkopf (1) einer endoskopischen Vorrichtung, der aufweist:
- eine Projektionsoptik (20-25, 9, 90), die dazu vorgesehen und ausgebildet ist, ein zu untersuchendes Objekt (3) mit Licht zu beleuchten, und
- eine Messoptik (4), die dazu vorgesehen und ausgebildet ist, vom zu untersuchenden Objekt (3) reflektiertes oder gestreutes Licht zu erfassen,
wobei die Projektionsoptik (20-25, 9, 90) Mittel (22, 25) umfasst oder der Projektionsoptik (20-25, 9, 90) Mittel des Messkopfes (1) zugeordnet sind, die ein Muster auf das zu untersuchende Objekt (3) projizieren, und die Messoptik (4) eine Aperturblende (5) aufweist, deren Apertur einstellbar ist,
**dadurch gekennzeichnet,**
**dass** eine kleinere Apertur zur Realisierung einer Inspektionsfunktion mit geringerer Auflösung und eine größere Apertur zur Realisierung einer Messfunktion mit höherer Auflösung einstellbar sind und im Fall der Messfunktion ein Muster auf das Objekt projizierbar ist, das im Fall der Inspektionsfunktion nicht oder zusätzlich abgebildet werden kann, wobei
(a) die Mittel ein Transmissionsgitter (22) mit unterschiedlichen Gitterkonstanten in unterschiedlichen Gitterbereichen aufweisen oder
(b) die Mittel mindestens zwei Monomode-Lichtwellenleiter (25) umfassen, deren Licht unter Bildung eines Gitters (7) miteinander interferiert, wobei der Abstand der Lichtwellenleiter (25) und/oder die Wellenlänge zur Einstellung der Gitterkonstanten einstellbar ist.

2. Messkopf nach Anspruch 1, **dadurch gekennzeichnet, dass** die Aperturblende (5) durch einen Farbfilter gebildet ist, wobei die Apertur des Farbfilters von der Lichtwellenlänge abhängt.

3. Messkopf nach Anspruch 2, **dadurch gekennzeichnet, dass** der Farbfilter mindestens zwei konzentrische Zonen (51-53) mit unterschiedlichen Durchlassspektren für Licht aufweist.

4. Messkopf nach einem der vorangehenden Ansprüche, **dadurch gekennzeichnet, dass** die Aperturblende (5) durch einen Polarisationsfilter gebildet ist, wobei die Apertur des Polarisationsfilters von der Polarisation des Lichtes abhängt.

5. Messkopf nach Anspruch 4, **dadurch gekennzeichnet, dass** der Polarisationsfilter mindestens zwei konzentrische Zonen (55-57) mit unterschiedlichen Polarisationsrichtungen aufweist.

6. Messkopf nach einem der vorangehenden Ansprüche, **dadurch gekennzeichnet, dass** die Aperturblende (5) mechanisch, elektrooptisch, elektrochromatisch, thermochromatisch oder durch schaltbare Flüssigkristalle im Hinblick auf die Apertur einstellbar ist.

7. Messkopf nach einem der vorangehenden Ansprüche, **dadurch gekennzeichnet, dass** der Messkopf (1) Mittel aufweist oder dem Messkopf Mittel zugeordnet sind, die Verzerrungen der Gitterlinie (223) auf dem zu untersuchenden Objekt (3) im Hinblick auf die durch diese Verzerrungen bereitgestellten 3D-Informationen auswerten.

8. Messkopf nach einem der vorangehenden Ansprüche, **dadurch gekennzeichnet, dass** die optischen Achsen von Projektionsoptik (20-25, 9, 90) und Messoptik (4) unter einem Winkel verlaufen.

9. Messkopf nach einem der vorangehenden Ansprüche, **dadurch gekennzeichnet, dass** die Projektionsoptik (21-24) derart ausgebildet ist, dass sie eine zentrierte Abbildung bereitstellt, oder dass die Projektionsoptik (21-24) derart ausgebildet ist, dass sie eine dezentrierte Abbildung bereitstellt.

10. Messkopf nach einem der vorangehenden Ansprüche, **dadurch gekennzeichnet, dass** die Projektionsoptik eine erste Beleuchtungsoptik (21-24) und eine zweite Beleuchtungsoptik (9, 90) aufweist, wobei die erste Beleuchtungsoptik (21-24) dazu ausgebildet und vorgesehen ist, Licht bereitzustellen, so dass die Aperturblende (5) eine erste Apertur realisiert, und die zweite Beleuchtungsoptik (9, 90) dazu ausgebildet und vorgesehen sind, Licht bereitzustellen, so dass die Aperturblende (5) eine zweite Apertur realisiert.

11. Messkopf nach einem der vorangehenden Ansprüche, **dadurch gekennzeichnet, dass** der Messkopf (1) des Weiteren einen Sensor (6) aufweist, der dazu vorgesehen und ausgebildet ist, das von der Messoptik (4) erzeugte Bild zu erfassen, und/oder der Messkopf (1) dazu vorgesehen und ausgebildet ist, Defekte in einem Flugzeugtriebwerk auszumessen.

12. Verfahren zur Inspektion und Messung eines Objektes unter Verwendung eines Endoskops mit einem Messkopf (1), der aufweist:
- eine Projektionsoptik (20-25, 9, 90), die dazu vorgesehen und ausgebildet ist, ein zu untersuchendes Objekt (3) mit Licht zu beleuchten, und
- eine Messoptik (4), die dazu vorgesehen und ausgebildet ist, vom zu untersuchenden Objekt (3) reflektiertes oder gestreutes Licht zu erfassen,
wobei die Projektionsoptik (20-25, 9, 90) Mittel (22, 25) umfasst oder der Projektionsoptik (20-25, 9, 90) Mittel des Messkopfes (1) zugeordnet sind, die ein Muster auf das zu untersuchende Objekt (3) projizieren, und die Messoptik (4) eine Aperturblende (5) aufweist, deren Apertur einstellbar ist,
**dadurch gekennzeichnet, dass**
eine kleinere Apertur zur Realisierung einer Inspektionsfunktion mit geringerer Auflösung und eine größere Apertur zur Realisierung einer Messfunktion mit höherer Auflösung eingestellt wird und im Fall der Messfunktion ein Muster auf das Objekt projiziert wird, das im Fall der Inspektionsfunktion nicht oder zusätzlich abgebildet wird, wobei
(a) die Mittel ein Transmissionsgitter mit unterschiedlichen Gitterkonstanten in unterschiedlichen Gitterbereichen aufweisen, oder
(b) die Mittel mindestens zwei Monomode-Lichtwellenleiter umfassen, deren Licht unter Bildung eines Gitters miteinander interferiert, wobei der Abstand der Lichtwellenleiter und/oder die Wellenlänge zur Einstellung der Gitterkonstanten einstellbar ist.

13. Verfahren nach Anspruch 12, **dadurch gekennzeichnet, dass** bei Verwendung der größeren Apertur Licht einer ersten Beleuchtungsoptik (21-24) zur Objektbeleuchtung und bei Verwendung der kleineren Apertur Licht einer zweiten Beleuchtungsoptik (9, 90) zur Objektbeleuchtung verwendet wird und/oder dass bei Verwendung der kleineren Apertur Lichtleitfaserbündel (9) zur Objektbeleuchtung eingesetzt werden.

14. Verfahren nach Anspruch 12 oder 13, **dadurch gekennzeichnet, dass** das Verfahren unter Verwendung eines Endoskops mit einem Messkopf (1) nach einem der Ansprüche 1 bis 11 durchgeführt wird.

## Claims

1. Measuring head (1) of an endoscopic apparatus, said measuring head comprising:
- a projection optics (20-25, 9, 90), which is provided and embodied to illuminate, with light, an object (3) to be examined, and
- a measuring optics (4), which is provided and embodied to capture light reflected or scattered by the object (3) to be examined,
wherein the projection optics (20-25, 9, 90) comprises means (22, 25) or means of the measuring head (1) are assigned to the projection optics (20-25, 9, 90), said means projecting a pattern onto the object (3) to be examined, and the measuring optics (4) has an aperture diaphragm (5) with an adjustable aperture,
**characterized**
**in that** a smaller aperture is able to be set in order to realize an inspection function with a lower resolution and a larger aperture is able to be set in order to realize a measurement function with a higher resolution and, in the case of the measurement function, a pattern is projectable onto the object, said pattern may not be imaged, or may be additionally imaged, in the case of the inspection function, wherein
(a) the means comprise a transmission grating (22) with different grating constants in different grating regions or
(b) the means comprise at least two monomode optical waveguides (25), the light of which interferes with one another under the formation of a grid (7), wherein the distance between the optical waveguides (25) and/or the wavelength is adjustable to set the grating constants.

2. Measuring head according to Claim 1, **characterized in that** the aperture diaphragm (5) is formed by a colour filter, wherein the aperture of the colour filter depends on the light wavelength.

3. Measuring head according to Claim 2, **characterized in that** the colour filter has at least two concentric zones (51-53) with different transmission spectra for light.

4. Measuring head according to any one of the preceding claims, **characterized in that** the aperture diaphragm (5) is formed by a polarization filter, wherein the aperture of the polarization filter depends on the polarization of the light.

5. Measuring head according to Claim 4, **characterized in that** the polarization filter has at least two concentric zones (55-57) with different polarization directions.

6. Measuring head according to any one of the preceding claims, **characterized in that** the aperture diaphragm (5) is adjustable in view of the aperture in mechanical, electro-optic, electrochromic or thermochromic fashion or by way of switchable liquid crystals.

7. Measuring head according to any one of the preceding claims, **characterized in that** the measuring head (1) comprises means or means are assigned to the measuring head, said means evaluating distortions of the grid line (223) on the object (3) to be examined in view of the 3-D information provided by these distortions.

8. Measuring head according to any one of the preceding claims, **characterized in that** the optical axes of the projection optics (20-25, 9, 90) and the measuring optics (4) are at an angle with respect to one another.

9. Measuring head according to any one of the preceding claims, **characterized in that** the projection optics (21-24) is embodied in such a way that it provides centred imaging or **in that** the projection optics (21-24) is embodied in such a way that it provides off-centred imaging.

10. Measuring head according to any one of the preceding claims, **characterized in that** the projection optics comprises a first illumination optics (21-24) and a second illumination optics (9, 90), wherein the first illumination optics (21-24) is embodied and provided to provide light so that the aperture diaphragm (5) realizes a first aperture and the second illumination optics (9, 90) is embodied and provided to provide light so that the aperture diaphragm (5) realizes a second aperture.

11. Measuring head according to any one of the preceding claims, **characterized in that** the measuring head (1) furthermore comprises a sensor (6), which is provided and embodied to capture the image generated by the measuring optics (4) and/or the measuring head (1) is provided and embodied to measure defects in an aircraft engine.

12. Method for inspecting and measuring an object using an endoscope with a measuring head (1), said measuring head comprising:
- a projection optics (20-25, 9, 90), which is provided and embodied to illuminate, with light, an object (3) to be examined, and
- a measuring optics (4), which is provided and embodied to capture light reflected or scattered by the object (3) to be examined,
wherein the projection optics (20-25, 9, 90) comprises means (22, 25) or means of the measuring head (1) are assigned to the projection optics (20-25, 9, 90), said means projecting a pattern onto the object (3) to be examined, and the measuring optics (4) has an aperture diaphragm (5) with an adjustable aperture,
**characterized in that**
a smaller aperture is able to be set in order to realize an inspection function with a lower resolution and a larger aperture is able to be set in order to realize a measurement function with a higher resolution and, in the case of the measurement function, a pattern is projected onto the object, said pattern may not be imaged, or may be additionally imaged, in the case of the inspection function, wherein
(a) the means comprise a transmission grating with different grating constants in different grating regions or
(b) the means comprise at least two monomode optical waveguides, the light of which interferes with one another under the formation of a grid, wherein the distance between the optical waveguides and/or the wavelength is adjustable to set the grating constants.

13. Method according to Claim 12, **characterized in that** light of a first illumination optics (21-24) is used for object illumination when the larger aperture is used and light of a second illumination optics (9, 90) is used for object illumination when the smaller aperture is used and/or **in that** optical fibre bundles (9) are used for the object illumination when using the smaller aperture.

14. Method according to Claim 12 or 13, **characterized in that** the method is carried out using an endoscope with a measuring head (1) according to any one of Claims 1 to 11.

## Revendications

1. Tête de mesure (1) d'un dispositif endoscopique, présentant:
- une optique de projection (20 à 25, 9, 90) qui est prévu et réalisé pour éclairer par la lumière un objet à examiner (3), et
- une optique de mesure (4) qui est prévu et réalisé pour capturer la lumière réfléchie ou diffusée par l'objet à examiner (3),
dans laquelle l'optique de projection (20 à 25, 9, 90) comprend des moyens (22, 25) ou des moyens de la tête de mesure (1) sont attribués au optique de projection (20 à 25, 9, 90) qui projettent un motif sur l'objet à examiner (3), et l'optique de mesure (4) présente un diaphragme d'ouverture (5) dont l'ouverture est réglable,
**caractérisée en ce qu'**il est possible de régler une ouverture plus petite pour réaliser une fonction d'inspection à résolution inférieure et de régler une ouverture plus grande pour réaliser une fonction de mesure à résolution supérieure, et dans le cas de la fonction de mesure, un motif peut être projeté sur l'objet qui ne peut pas être représenté ou peut être représenté en plus dans le cas de la fonction d'inspection, dans laquelle
(a) les moyens présentent un réseau de transmission (22) ayant différentes constantes de réseau dans différentes zones de réseau, ou
(b) les moyens comprennent au moins deux guides d'ondes optiques monomodes (25) dont la lumière interfère mutuellement en formant un réseau (7), l'espacement des guides d'ondes optiques (25) et/ou la longueur d'onde étant réglable(s) pour régler les constantes de réseau.

2. Tête de mesure selon la revendication 1, **caractérisée en ce que** le diaphragme d'ouverture (5) est formé par un filtre de couleur, dans lequel l'ouverture du filtre de couleur dépendant de la longueur d'onde de la lumière.

3. Tête de mesure selon la revendication 2, **caractérisée en ce que** le filtre de couleur présente au moins deux zones concentriques (51 à 53) ayant des spectres de transmission différents pour la lumière.

4. Tête de mesure selon l'une quelconque des revendications précédentes, **caractérisée en ce que** le diaphragme d'ouverture (5) est formé par un filtre de polarisation, et dans lequel l'ouverture du filtre de polarisation dépendant de la polarisation de la lumière.

5. Tête de mesure selon la revendication 4, **caractérisée en ce que** le filtre de polarisation présente au moins deux zones concentriques (55 à 57) ayant des directions de polarisation différentes.

6. Tête de mesure selon l'une quelconque des revendications précédentes, **caractérisée en ce que** le diaphragme d'ouverture (5) est réglable en ouverture de manière mécanique, électro-optique, électrochromatique, thermochromatique ou par des cristaux liquides commutables.

7. Tête de mesure selon l'une quelconque des revendications précédentes, **caractérisée en ce que** la tête de mesure (1) présente des moyens ou **en ce que** des moyens sont attribués à la tête de mesure qui évaluent des distorsions de la ligne de réseau (223) sur l'objet à examiner (3) en ce qui concerne les informations 3D fournies par ces distorsions.

8. Tête de mesure selon l'une quelconque des revendications précédentes, **caractérisée en ce que** les axes optiques de l'optique de projection (20 à 25, 9, 90) et de l'optique de mesure (4) s'étendent selon un angle.

9. Tête de mesure selon l'une quelconque des revendications précédentes, **caractérisée en ce que** l'optique de projection (21 à 24) est réalisé de telle sorte qu'il fournit une représentation centrée, ou **en ce que** l'optique de projection (21 à 24) est réalisé de telle sorte qu'il fournit une représentation décentrée.

10. Tête de mesure selon l'une quelconque des revendications précédentes, **caractérisée en ce que** l'optique de projection présente un premier optique d'éclairage (21 à 24) et un deuxième optique d'éclairage (9, 90), le premier optique d'éclairage (21 à 24) étant réalisé et prévu pour fournir de la lumière de sorte que le diaphragme d'ouverture (5) réalise une première ouverture, et le deuxième optique d'éclairage (9, 90) étant réalisé et prévu pour fournir de la lumière de sorte que le diaphragme d'ouverture (5) réalise une deuxième ouverture.

11. Tête de mesure selon l'une quelconque des revendications précédentes, **caractérisée en ce que** la tête de mesure (1) présente en outre un capteur (6) qui est prévu et réalisé pour capturer l'image produite par l'optique de mesure (4) et/ou la tête de mesure (1) est prévue et réalisée pour mesurer des défauts dans un moteur d'avion.

12. Procédé permettant d'inspecter et de mesurer un objet en utilisant un endoscope doté d'une tête de mesure (1), présentant:
- une optique de projection (20 à 25, 9, 90) qui est prévu et réalisé pour éclairer par la lumière un objet à examiner (3), et
- une optique de mesure (4) qui est prévu et réalisé pour capturer la lumière réfléchie ou diffusée par l'objet à examiner (3),
dans lequel l'optique de projection (20 à 25, 9, 90) comprend des moyens (22, 25) ou des moyens de la tête de mesure (1) sont attribués au optique de projection (20 à 25, 9, 90) qui projettent un motif sur l'objet à examiner (3), et l'optique de mesure (4) présente un diaphragme d'ouverture (5) dont l'ouverture est réglable,
**caractérisé en ce qu'**une ouverture plus petite pour réaliser une fonction d'inspection à résolution inférieure est réglée et une ouverture plus grande pour réaliser une fonction de mesure à résolution supérieure est réglée, et dans le cas de la fonction de mesure, un motif est projeté sur l'objet qui n'est pas représenté ou est représenté en plus dans le cas de la fonction d'inspection, dans lequel
(a) les moyens présentent un réseau de transmission ayant différentes constantes de réseau dans différentes zones de réseau, ou
(b) les moyens comprennent au moins deux guides d'ondes optiques monomodes dont la lumière interfère mutuellement en formant un réseau, l'espacement des guides d'ondes optiques et/ou la longueur d'onde étant réglable(s) pour régler les constantes de réseau.

13. Procédé selon la revendication 12, **caractérisé en ce que** lors de l'utilisation de l'ouverture plus grande, la lumière d'un premier système d'éclairage (21 à 24) est utilisée pour éclairer l'objet, et lors de l'utilisation de l'ouverture plus petite, la lumière d'un deuxième système d'éclairage (9, 90) est utilisée pour éclairer l'objet, et/ou **en ce que** lors de l'utilisation de l'ouverture plus petite, des faisceaux de fibres optiques (9) sont mis en œuvre pour éclairer l'objet.

14. Procédé selon la revendication 12 ou 13, **caractérisé en ce que** le procédé est exécuté en utilisant un endoscope doté d'une tête de mesure (1) selon l'une quelconque des revendications 1 à 11.
